# EUROPEAN PATENT SPECIFICATION

(11) **EP 1 519 697 B1**
(45) Date of publication and mention of the grant of the patent: **18.11.2015**
(21) Application number: 03762619.9
(22) Date of filing: 04.07.2003
(51) Int. Cl.: A61F 2/95, A61F 2/966

(54) **STENT DELIVERY SYSTEM**
STENT-ABLAGESYSTEM
SYSTEME D'APPORT DE STENT

(30) Priority: 05.07.2002 US 189993
(43) Date of publication of application: 06.04.2005
(60) Divisional application: 10181967.0
(73) Proprietor: C.R. Bard, Inc., Murray Hill, NJ 07974 (US)
(72) Inventor: BUCK, Jerry, C., Coconut Creek, FL 33073 (US); KRATSCH, Peter, K., Sunrise, FL 33323 (US); FULKERSON, John, D., Plantation, FL 33322 (US); SLATER, Charles, R., Fort Lauderdale, FL 33312 (US); GOTTLIEB, Saul, Miramar, FL 33027 (US)
(74) Representative: Hoffmann Eitle
(86) International application number: PCT/EP2003/007169
(87) International publication number: WO 2004/004597

(56) References cited:
- EP-A1- 1 380 271
- EP-A2- 0 747 021
- WO-A2-02/102279
- DE-U1- 29 717 110

## Description

### 1. Field of the Invention

This invention relates broadly to medical devices. More particularly, this invention relates to an instrument for delivering a self-expanding vascular stent into a mammalian body and controllably releasing the stent.

### 2. State of the Art

Transluminal prostheses are widely used in the medical arts for implantation in blood vessels, biliary ducts, or other similar organs of the living body. These prostheses are commonly known as stents and are used to maintain, open, or dilate tubular anatomical structures.

Stents are either balloon expandable or self-expanding. Balloon expandable stents are typically made from a solid tube of stainless steel.
Thereafter, a series of cuts are made in the wall of the stent. The stent has a first smaller diameter configuration which permits the stent to be delivered through the human vasculature by being crimped onto a balloon catheter. The stent also has a second, expanded diameter configuration, upon the application, by the balloon catheter, from the interior of the tubular shaped member of a radially, outwardly directed force.

Self-expanding stents act like springs and recover to their expanded or implanted configuration after being compressed. As such, the stent is inserted into a blood vessel in a compressed state and then released at a site to deploy into an expanded state. One type of self-expanding stent is composed of a plurality of individually rigid but flexible and elastic thread elements defining a radially self-expanding helix. This type of stent is known in the art as a "braided stent". Placement of such stents in a body vessel can be achieved by a device which comprises an outer catheter for holding the stent at its distal end, and an inner piston which pushes the stent forward once it is in position. However, braided stents have the disadvantage that they typically do not have the necessary radial strength to effectively hold open a diseased vessel. In addition, the plurality of wires or fibers used to make such stents could become dangerous if separated from the body of the stent, where it could pierce through the vessel.

Therefore, recently, self-expanding stents cut from a tube of superelastic metal, e.g., a nickel-titanium alloy, have been manufactured. These stents are crush recoverable and have relatively high radial strength. To enhance the radiopacity of surgical stents, one or more radiopaque markers may be provided on the stent which is clearly identifiable when a fluoroscope or other imaging device is used.

Stents are delivered to an implant site with the use of a delivery system. Delivery systems for self-expanding stents generally comprise an inner tubular member on which the stent is loaded and which may be fed over a guidewire, and an outer tubular member or jacket longitudinally slidable over the inner tubular member and adapted to extend over the stent during delivery to the implant site. The jacket is retracted along the inner tubular member to release the self-expanding stent from the inner tubular member.

In several available delivery systems, the jacket and inner member are freely movable relative to each other and must be separately manually held in the hands of the physician. After the distal end of the system is located at the implant site, the inner member must be held still to prevent dislocation. However, it is very difficult to maintain the position of the inner member while moving the outer member to deploy the stent. As such, the degree of control during deployment is limited. Under such limited control there is a tendency for the stent to escape from the inner member before the jacket is fully retracted and jump from the desired deployment site. This may result in deployment of the stent at a location other than the desired implant site.

A handle may be provided to move the outer tubular member relative to the inner tubular member with greater control. For example, Medtronic Inc., utilizes a handle which can lock the inner tube and outer jacket relative to each other and effect relative movement of the two to cause deployment of the stent. However, such handles have several shortcomings. First, the handle is not particularly well suited to short stents as there is little fine control. Second, the handle is not well-suited to long stents, e.g., up to 90 mm in length, as the linear control requires the operator to change his or her grip during deployment in order to generate the large relative motion of the tubular components. Third, it is possible for the stent to automatically release before the jacket is fully retracted from over the stent. This is because the superelastic expansion of the stent causes the stent to slip distally out of the deployment system before the operator retracts the sheath. The result can be an unintentionally rapid and possibly uneven deployment of the stent. Fourth, without reference to a fluoroscope monitoring the stent, there is no manner to determine from the proximal end of the instrument the progress of stent deployment. Fifth, the construction of the inner tubular member and outer jacket may cause the inner member and jacket to be crushed during use. Furthermore, the inner tubular member is subject to compressive force during deployment and may deform while moving the stent from the desired deployment location.

A thumb wheel is a common way to deploy the stent from the handle. See for example DE-U1-29717110, EP-A2-747021, EP-A1-1380271 and WO-A1-01/56504.

### SUMMARY OF THE INVENTION

It is an object of the invention to provide a stent delivery system that permits a high degree of control during deployment of the stent.

It is another object of the invention to provide a stent delivery system which can be operated with a single hand.

It is an additional object of the invention to provide a stent delivery system which is adapted for use with stents of various lengths.

The invention is defined in claim 1 below. The dependent claims are directed to optional features and preferred embodiments.
The invention provides a stent delivery system, comprising: an inner tubular member having a proximal end and a distal end; a stent loaded on said distal end of said inner tubular member; an outer jacket longitudinally slidable over said inner tubular member and having a proximal end and distal end; and a handle including a stationary member, a movable member, and a mechanism for moving the movable member relative to the stationary member; the mechanism comprising two knobs rotatably coupled to the stationary member and mechanically coupled to said movable member such that rotation of at least one said knob causes movement of said movable member relative to said stationary member; wherein said proximal end of said inner tubular member is fixedly coupled to said stationary member and said proximal end of said outer jacket is fixedly coupled to said movable member.

In accord with preferred aspects of the invention, the stationary member is preferably elongate and adapted to ergonomically fit in either a physician's left or right hand. The movable member is fixed to a belt extending about two sprockets, and one of the sprockets is coupled preferably via one or more gears to the knobs located on both sides of the handle. The knobs are situated such that when the handle is held in a hand, one of the knobs may be rotated by the thumb of the same hand of the physician holding the handle to effect single-handed longitudinal movement of the outer jacket relative to the inner tubular member. The gears used in the handle can be chosen to effect more or less longitudinal travel of the outer jacket relative to a given rotational movement of the knobs. That is, the handle can be adapted to conveniently deploy stents of various lengths with a common rotational movement of the knob relative to the handle. The handle also includes a mechanism which produces an audible click as the knob is rotated to provide audible feedback to the physician regarding movement of the outer jacket.

In accord with another preferred aspect of the invention, the proximal portion of the outer jacket is provided with incremental visual indicia. The visual indicia preferably correspond to the length of the stent being deployed. As such, as the jacket is retracted from the inner tubular member and into the handle, the indicia can be seen to move relative to the strain relief. The jacket can also be provided with relief to provide tactile feedback to the physician.

In accord with other preferred aspects of the invention, the inner tubular member and outer jacket are each preferably substantially trilayer constructions. Each preferably includes an inner layer, a middle layer including a flat wire braid, and an outer layer. The trilayer construction provides a combination of flexibility and columnar strength. The inner tubular member includes a reduced diameter portion on which the stent is loaded. A shoulder is defined at the transition of the inner tubular member into its reduced diameter portion, and the shoulder functions as a stop for the stent. The reduced diameter portion also preferably includes a protruding formation adjacent the shoulder. The formation operates to clamp a proximal end of the stent between the inner tubular member and the outer jacket and thereby secure the stent on the inner tubular member until the outer jacket is fully retracted from over the stent.

As such, the stent deployment device provides greater control over stent deployment via visual and auditory feedback at the proximal end of the instrument, increased control of the relative movement of the outer jacket relative to the inner tubular member, and prevention of premature release of the stent from the deployment device.

Additional objects and advantages of the invention will become apparent to those skilled in the art upon reference to the detailed description taken in conjunction with the provided figures.

### BRIEF DESCRIPTION OF THE DRAWINGS

Fig. 1 is a perspective view of the stent delivery system according to the invention;
Fig. 2 is a side elevation view of the stent delivery system according to the invention;
Fig. 3 is a schematic cross-section view of the distal end of the stent delivery system according to the invention;
Fig. 4 is a perspective view of a proximal handle portion of the stent delivery system, with one half of the stationary member, a knob cover, the inner tubular member, the outer jacket, the rear sprocket, and the belt removed;
Fig. 5 is a side elevation view of a proximal handle portion of the stent delivery system, with one half of the stationary member and a knob cover removed;
Fig. 6 is a schematic top view of a proximal portion of the outer jacket and the strain relief sleeve of the stent delivery system;
Fig. 7 is a perspective view of a cradle for supporting a handle of the stent delivery system;
Fig. 8 is a perspective view of the cradle of Fig. 7 shown supporting the handle of the stent delivery system;
Fig. 9 is a perspective view of a handle provided with a
   keyed locking system, shown with the key inserted in the keyhole in a locked configuration, which when in the locked configuration prevents movement of the knobs relative to the stationary member;
Fig. 10 is a perspective view of a handle provided with a
   keyed locking system, shown with the key removed from the keyhole in an unlocked configuration;
FIG. 11 is a section view transverse through the stationary member and knobs of the handle and a side elevation of a clip which when in a locked configuration with the handle provide a lock which prevents movement of the knobs relative to the stationary member, the system shown in an unlocked configuration; and
FIG. 12 is a perspective section view of the stationary member and knobs of the handle and the clip in the locked configuration.

### DETAILED DESCRIPTION OF THE PREFERRED EMBODIMENTS

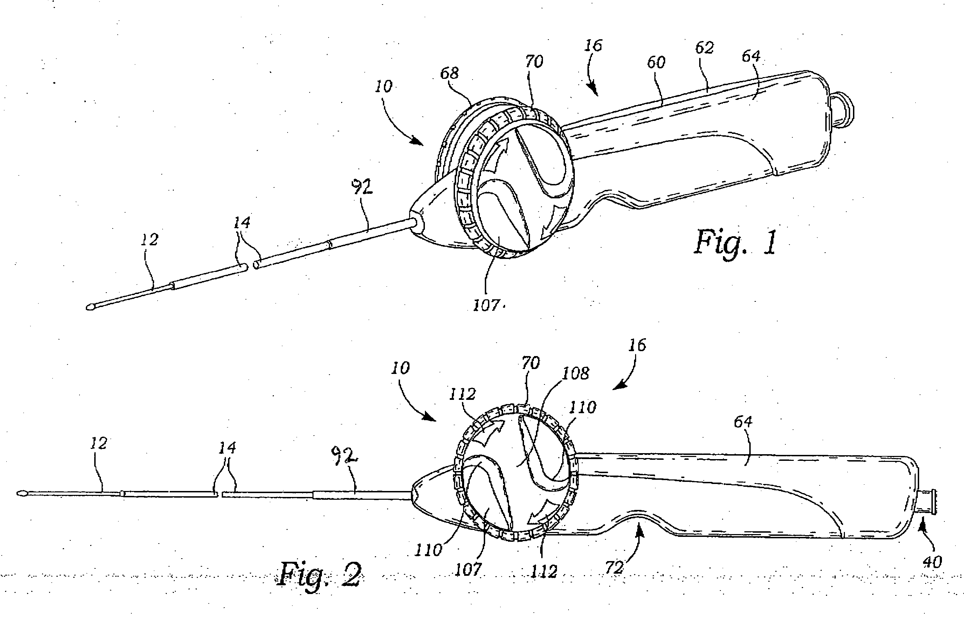

Referring now to FIGS. 1 and 2, a stent delivery system 10 generally includes an inner tubular member 12, a tubular jacket 14 slidable over the inner tubular member 12, and a handle 16 adapted to effect longitudinal movement of the jacket 14 relative to the inner tubular member 12.

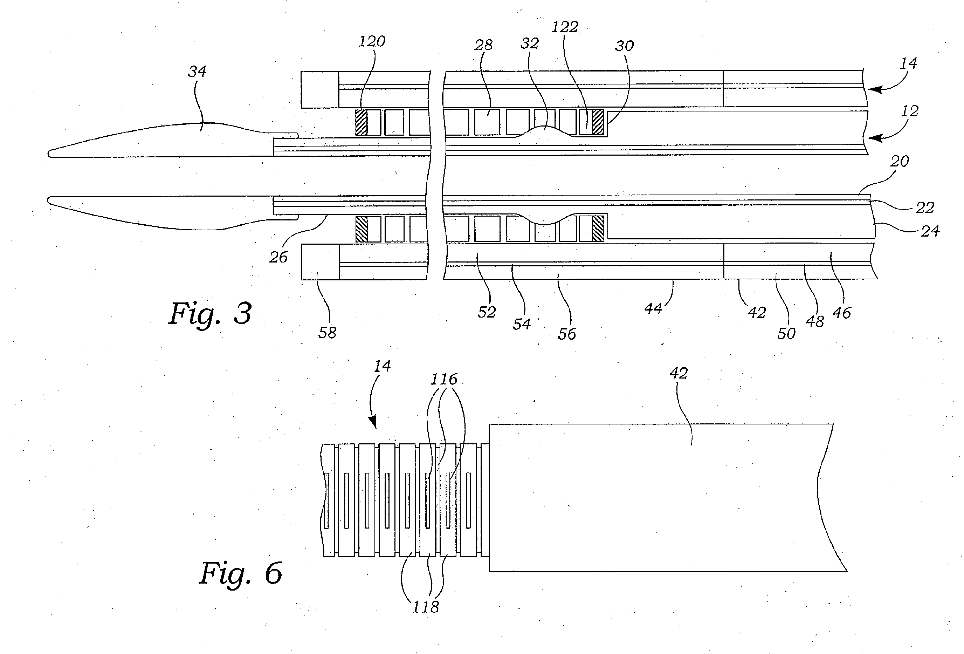

Turning now to FIG. 3, the inner tubular member 12 is preferably a coextruded, trilayer construction. The inner layer 20 is preferably polytetrafluoroethylene (PTFE), fluorinated ethylene propylene (FEP), high density polyethylene (HDPE), or urethane. The middle layer 22 is a wire braid, and more preferably a 304V stainless steel flat wire braid of 1x3 (40 picks) construction, with wires having a 25.4µm by 76.2pm (0.001 inch by 0.003 inch) rectangular cross-section. Wires of other metals and alloys may also be used, including other stainless steel alloys, cobalt-chrome alloys, and other high-strength, high-stiffness, corrosion-resistant metal alloys. The outer layer 24 is preferably a thermoplastic, melt processible, polyether-based polyamide, such as PEBAX®-7033 available from Modified Polymer Components, Inc. of Sunnyvale, CA. In the extrusion process, the inner and outer layers are bonded to each other and encapsulate the metallic reinforcing middle wire layer to create an integrated tubing. This tubing exhibits high lateral flexibility combined with a high degree of longitudinal stiffness (resistance to shortening), and also high torqueability. Thus, the inner tubular member is very controllable.

A distal portion 26 of the inner tubular member 12, at the location where a stent 28 is loaded, is reduced in diameter, e.g., via centerless grinding, laser grinding, or thermal reduction of the outer layer 24. A shoulder 30 is defined at the transition of the inner tubular member into its reduced diameter distal portion. The shoulder 30 functions as a stop for the stent to prevent the stent from moving proximally on the inner tubular member 12 when the jacket 14 is retracted. The reduced diameter portion also preferably includes a narrow preferably circumferential ridge 32 adjacent the shoulder 30. The proximal end of the stent is frictionally engaged by compression between the ridge of the inner member and the outer sheath. As a result, the stent is prevented from self-advancing out of the delivery system until that ridge of the inner member has been uncovered by the proximally-retracting outer jacket. The distalmost end of the inner tubular member is preferably provided with a tubular soft flexible radiopaque tip 34.

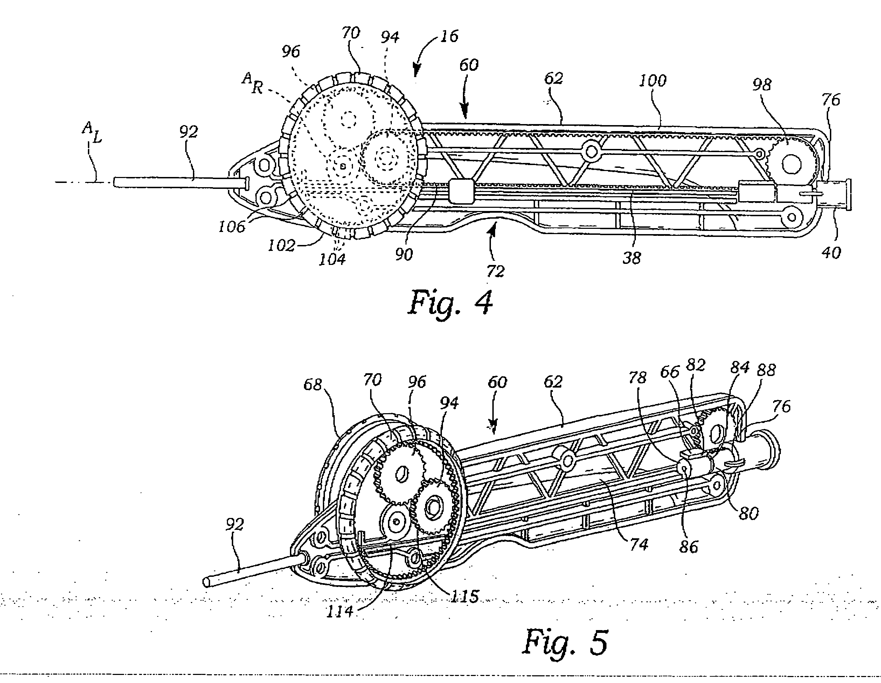

Referring to Figs. 2 and 4, a proximal end of the inner tubular member 12 is coupled, e.g., via bonding, to a longitudinally stiff, preferably stainless steel tube 38 of substantially the same outer diameter. The proximal end of the stiff tube 38 is provided with a luer adapter 40 permitting convenient coupling to a mating luer connection and facilitating flushing of the inner tubular member.

Turning back to Fig. 3, the outer jacket 14 includes a first portion 42 extending from its proximal end to near the distal end which preferably has the same trilayer construction as the inner tubular member, and preferably a second portion 44 of a different construction adjacent at its distal end. That is, the first portion 42 has an inner layer 46 that is preferably PTFE, FEP, HDPE or urethane, a middle layer 48 that is a preferably stainless steel flat wire braid construction, and an outer layer 50 that is preferably a thermoplastic, melt processible, polyether-based polyamide. The second portion 44 of the outer jacket 14 is preferably a trilayer coextrusion having an inner layer 52 preferably of PTFE, FEP, HDPE or urethane, a middle tie-layer polymer resin 54, such as PLEXAR® available from Equistar Chemicals, LP of Clinton, IA, and an outer layer 56 of a thermoplastic, melt processible, polyether-based polyamide. The middle tie-layer resin 54 permits the inner and outer layers 52, 56 to be bonded together into a co-extruded or multilayer composition. The second portion 44 of the outer jacket preferably does not include a braided middle layer, and thus has increased flexibility. In addition, the second portion 44 is preferably a clear construction, permitting visible observation of the stent loaded on the distal portion of the inner tubular member. The first and second portions 42, 44 are preferably substantially seamlessly coupled together using bonding, coextrusion, or other means known in the art; i.e., there are no imperfections at the junction thereof which would interfere with smoothly retracting the outer jacket over the inner tubular member. The distal end of the second portion 44 preferably includes a radiopaque marker 58, such that under fluoroscopy the location of distal end of the jacket relative to fluoroscopically-visible elements of the loaded stent can be monitored. The marker 58 is preferably constructed of a radiopaque metallic material so that it may be crimped securely to the outer jacket. Exemplar suitable materials include platinum, platinum-iridium alloy, tantalum, tantalum-tungsten alloy, zirconium alloy, gold, gold alloy, and palladium, all of which are well-known for use as radiopaque markers in catheter devices.

Referring to Fig. 1, 2, 4 and 5, the handle 16 generally includes an elongate stationary member 60 defined by two shells portions 62, 64, an internal longitudinally movable member 66, and a pair of manually rotatable wheel-like knobs 68, 70 which effect movement of the movable member 66 relative to the stationary member 60, as described in more detail below.

More particularly, the exterior of the stationary member 60 is preferably ergonomically shaped to fit in the palm of either a left or right hand of an operator and includes a lower grip 72 permitting a pointer finger of the hand of the operator to secure the handle in the palm of the hand. The interior of the stationary member defines an axial track 74 and a rear opening 76. The movable member 66 has a preferably substantially cruciate cross-sectional shape, with lateral portions 78, 80 residing in the track defined by the shell portions 62, 64 of the stationary member 60. An upper portion 82 of the movable member 66 defines a toothed slot 84, and an axial throughbore 86 is provided through a central portion of the movable member.

Referring to Fig. 4, the stiff tubular portion 38 at the proximal end of the inner tubular member 12 extends through the axial throughbore 86, and a portion of the luer connection 76 is coupled in a pocket 88 (Fig. 5) at the rear end of the stationary member 60 such that the luer connection extends from the rear of the stationary member. As such, the inner tubular member 12 is longitudinally fixed relative to the handle 16, and the stiff tubular portion 38 provides very high longitudinal stiffness at the proximal end of the inner tubular member. On the other hand, the outer jacket 14 has a proximal end 90 which is fixedly coupled in the axial throughbore 86 of the movable member 66. Thus, the outer jacket 14 moves relative to the stationary member 60 of the handle 16. A strain relief sleeve 92 is fixed to the stationary member 60 and extends distally from the stationary member. The outer jacket 14 is therefore likewise movable relative to the strain relief sleeve 92.

In addition, the stationary member 60 is provided at its distal end with a first rotating sprocket 94, and a gear 96 coupled to the first sprocket 94, and at its proximal end with a second rotating sprocket 98. A toothed belt 100 extends around the first and second sprockets 94, 98. A portion of the belt is provided in the toothed slot 84 of the movable member 66 to thereby lock the movable member to the belt. As a result, rotation of the gear 96 causes movement of the belt, which results in movement of the moveable member 66 and movement of the outer jacket 14 relative to the handle 16 and the inner tubular member 12.

The knobs 68, 70 are provided one of each side of the stationary member 60 and connected together with a screw (not shown). The knobs 68, 70 are rotatable relative to the stationary member 60, preferably with the axis of rotation A_{R} being vertically offset above the longitudinal axis A_{L} of the stent delivery system 10. Due to the offset of the axis of rotation A_{R} relative to the longitudinal axis A_{L}, the knobs 68, 70 can be kept to a comfortable relatively small size while permitting an upper portion of each knob to rise above the top of the stationary member of the handle. As a result, when the handle 16 is held in either the left or right hand of the physician, the thumb of that hand is situated for placement on one of the knobs. One of the knobs, e.g., knob 70, includes a peripheral portion 102 provided with inwardly-directed gear teeth 104 that engage the gear 96, and a knob cover 106. The circumference of the peripheral portion 102 of each knob is preferably entirely exposed (i.e., located outside the stationary member 60) and provided with a friction-enhancing material such as rubber in which is provided a finger engagement structure, such as grooves 106, ribs, or knurls. The respective knob 68, 70 may then be easily rotated by movement of the physician's thumb to effect retraction of the jacket 14 from over the inner tubular member 12. As such, the instrument is adapted for single-handed operation by either hand of the physician.

Nevertheless, it may be desirable by some operators to operate the handle 16 with two hands, one holding the stationary member 60 and the other rotating one of the knobs 68, 70. Therefore, referring to Fig. 2, in order to facilitate this manner of operation, the cover portion 107 of each knob is formed with a raised substantially diametric grip 108 and includes contours 110 adapted to receive a distal portion of thumb to provide leverage in turning the knob. This structure also implicitly identifies the direction of knob rotation for jacket retraction. Moreover, each knob is preferably provided with arrows 112 which explicitly indicate the direction of required rotation.

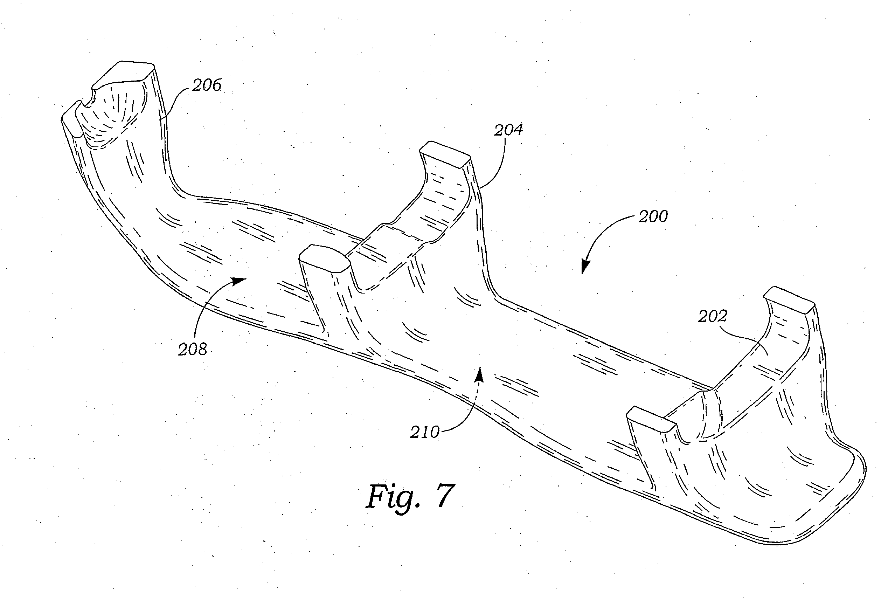

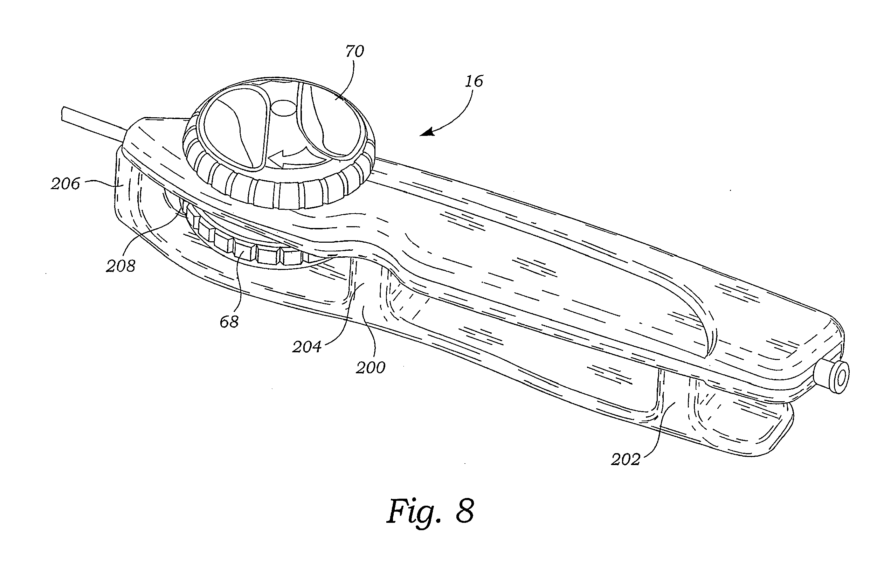

Furthermore, it may be desired by some operators of the instrument to stabilize the handle on a platform, such as the operating table. In accord therewith, referring to FIGS. 7 and 8, a cradle 200 is provided. The cradle 200 includes supports 202, 204, 206 which are adapted to stably hold the handle 16 on its side. When held by the cradle 200, one knob 68 of the handle is received in a space 208, and the other knob 70 faces upward. Knob 68 is positioned in the space 208 such that it freely rotates when knob 70 is manually rotated. The bottom surface 210 of the cradle 200 may be coupled to a platform, e.g., with double-sided adhesive tape. With the handle 16 supported on the cradle 200, the operator may stabilize the handle on the cradle with a hand, and rotate knob 70 to effect stent deployment.

The handle can be adapted with sprockets and gears having different sizes and different numbers of gear teeth, and knobs of different diameters. In this manner, the motion by the operator's hand and corresponding motion of the distal components of the delivery system is adjustable so that the delivery instrument is optimized for each length of stent. Accordingly, the same amount of hand motion by the operator may be translated into relatively less motion in a delivery instrument on which a short stent is loaded, and translated into relatively more motion in a delivery instrument on which a longer stent is loaded. Thus, preferably a common rotational movement may be utilized to deploy stents of any length. In addition, a gear system may be employed with a suitable operator-engageable extra step-down gear that permits the operator to choose between gear ratios that provide enhanced control for short or longer stents.

A stiffly resilient element, e.g., a metal leaf spring 114 (FIG. 5), is also provided in the stationary member 60 and has an end 115 which is in contact with the first sprocket 94. As the first sprocket is rotated, the teeth thereof successively contact the resilient element and produce an audible clicking sound, providing feedback to the physician or other operator that the rotation of the knobs is causing operation of a mechanism at the interior of the handle. In addition, the location of the spring 114 relative to the first sprocket 94 prevents rotation of the handles in a direction which would cause movement of the outer jacket distally over the inner tubular member. Thus, the operator is prevented from attempting to retract the stent back into the outer jacket, as most self-expanding stent designs do not allow such retraction, and the stent would be damaged thereby.

The proximal portion of the outer jacket is provided with incremental or quantitative visual indicia 116 (FIG. 6). The visual indicia preferably correspond to the length of the stent being deployed. As such, as the outer jacket 14 is retracted from over the inner tubular member 12 and into the strain relief handle, the indicia can be seen to move relative to the strain relief sleeve 92, and the operator can determine from inspection at the proximal end of the instrument how much of the stent remains to be deployed. The visual indicia may extend only the length of the stent loaded in the system, or may extend the maximum length of any stent which may be loaded on the system, and include discrete markings to indicate the jacket retraction required for deployment of stents of various lengths, e.g., markings at 15 mm, 30 mm, 60 mm, and 90 mm. In addition, the proximal end of the outer jacket may be provided with relief 118, either recessed beneath the surface (as shown) or protruding from the surface, so that the operator may also determine the degree of deployment by tactile feel. The tactile indicia may be coincident or independent of the visual indicia.

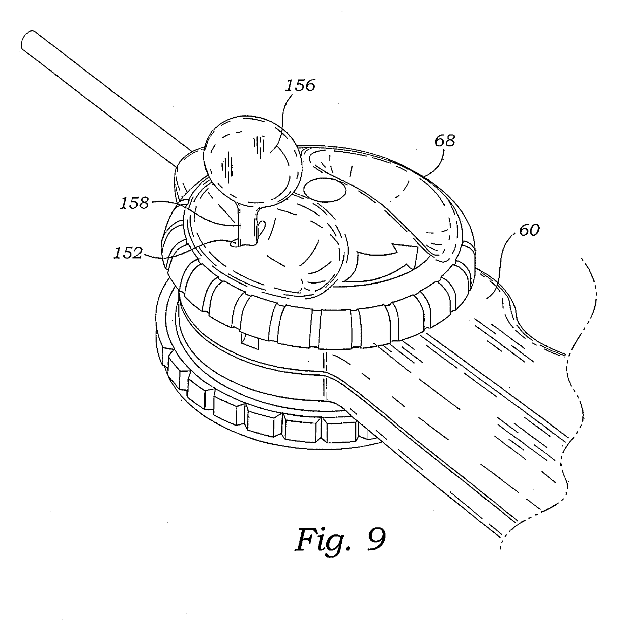

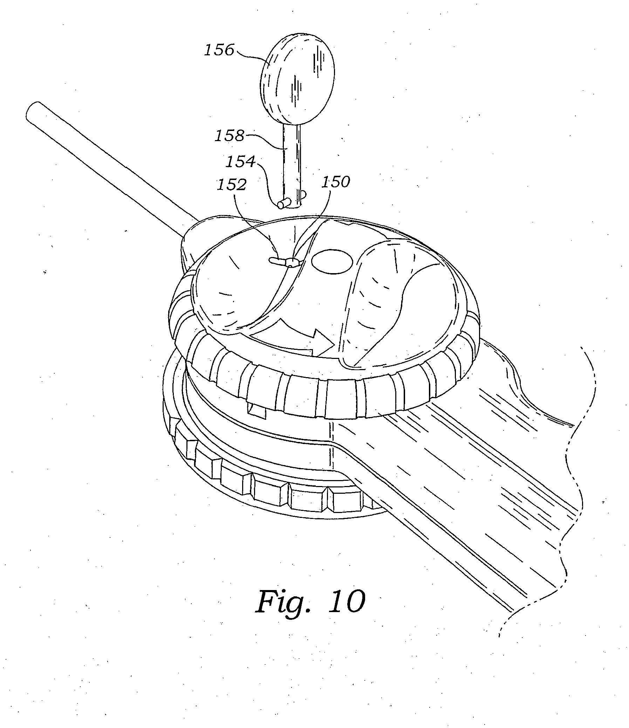

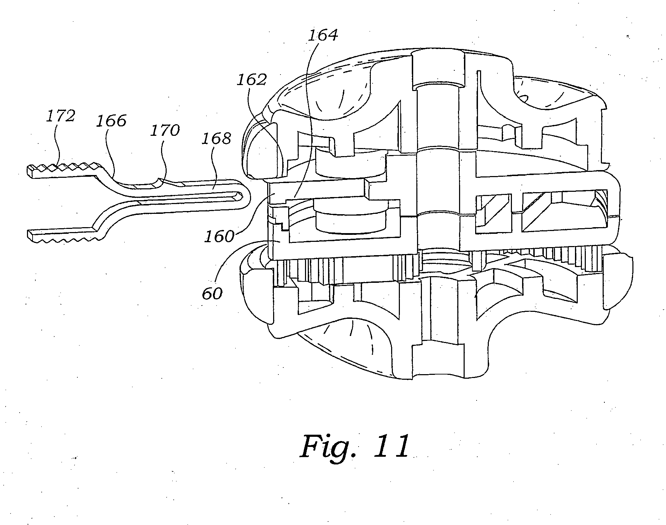

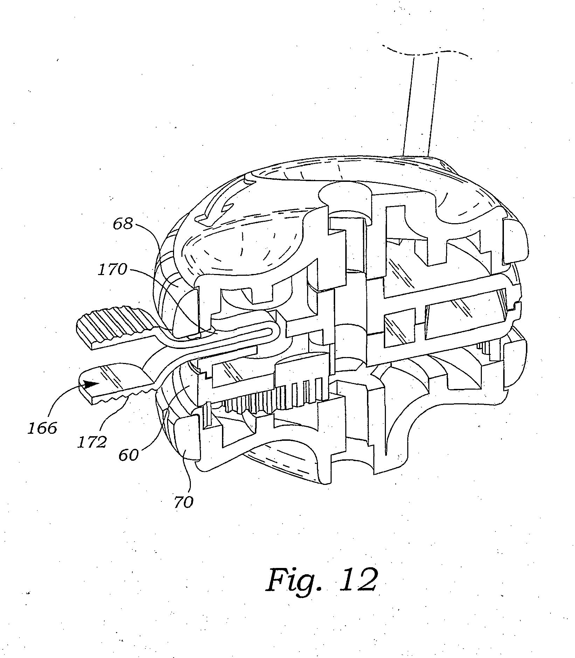

A locking system is provided to prevent movement of the belt until the system is unlock. Referring to FIG. 9, knob 68 and the stationary member 60 of the handle 16 each include a keyhole which preferably extends parallel to the axis of rotation A_{R} of the knobs 68, 70 (Fig. 4). The keyhole 150 in the knob 68 includes a slot 152 which is preferably oriented substantially transverse to a slot (not shown) in the stationary member 60; i.e., the slot in the stationary member 60 is in the same orientation as the crossbar 154 on the shaft 158 of the key 156 shown in Fig. 10. When the key 156 is fully inserted into the keyhole, the key interferes with rotation of knob 68. As such, the key 156 prevents inadvertent partial or full deployment of the stent while the key is in place; i.e., during shipping and storage of the stent-loaded instrument. When the instrument 10 is prepared for use, the key 150 can be turned and withdrawn (Fig. 10). Other suitable locking mechanisms can also be used. By way of another example, referring to Figs. 11 and 12, a lower side of the stationary member 60 is provided with an opening 160, and knob 68 includes a notch 162 which when aligned adjacent the opening 160 defines a channel 164 for receiving a spring clip 166. A spring clip 166 includes a resilient U-shaped portion 168 having a barb 170 along one side thereof, and a handle 172 permitting the U-shaped portion 168 to be manually reduced in dimension. When the knob 68 is aligned relative to the opening 160 to provide access to the channel 164, the U-shaped portion 168 can be placed in the channel 164 with the U-shaped portion 168 being compressed as the barb 170 contacts the area about the opening 160. The U-shaped portion 168 springs back to shape once in the stationary member 60, as the barb 170 seats in the notch 162 (Fig. 12). The barb 170 interferes with rotation of the knob 68, and thus locks the knobs 68, 70 relative to the stationary member 60. When it is desired to use the device, the clip handle 172 is compressed and the clip 166 is removed.

In use, with the locking system unlocked, and the distal end of the inner tubular member is fed over a guidewire and guided therealong to the deployment site. The distal end of the delivery instrument is then fluoroscopically viewed to ascertain that the instrument is in a predeployment configuration. That is, the delivery instrument is optimized for use with self-expanding stents having a plurality of radiopaque markers 120, 122 at each of its ends, and the ends of the stent are seen to be situated proximal of both the radiopaque tip 34 of the inner tubular member 12 and the radiopaque marker 58 at the distal end of the outer jacket 14 (Fig. 3). One or both of the knobs 68, 70 on the handle 16 is/are then manually rotated relative to the handle to cause retraction of the outer jacket 14. The handle preferably provides audible, tactile, and visual indications of the retraction. Under fluoroscopy, the marker 58 on the jacket 14 is seen to move proximally toward and past the distal stent markers 120. As the stent exits the distal end of the catheter, the distal stent markers 120 are seen to separate radially as the stent 28 self-expands. When the jacket 14 is fully retracted from over the stent 14, the clamping force (created by clamping the proximal end of the stent between the protruding ring 32 on the inner tubular member 12 and the interior of the outer jacket 14) is removed from the proximal end of the stent. When the stent 28 is completely released, the markers 120, 122 at both ends of the stent are seen to be expanded radially, and the marker 58 on the outer jacket is positioned proximal to the markers 122 on the proximal end of the stent.

From the foregoing, it is appreciated that the stent delivery system provides greater control over stent deployment via one or more visual and auditory feedback at the proximal end of the instrument, increased control of the relative movement of the outer jacket relative to the inner tubular member, and prevention of premature release of the stent from the deployment instrument.

While particular preferred trilayer constructions for the inner tubular member and outer jacket have been disclosed, it will be appreciated that other constructions, of single or multiple layers and of other materials can be used as well. In addition, while a particular handle configuration has been disclosed, it will be understood that other handles, preferably which permit single-handed operation can also be used. For example, a lower portion of the knobs may be housed within the handle with only a top portion exposed for actuation by an operator's thumb. Furthermore, various features of the illustrated embodiments can be used alone without the use of other features. For example, the construction of the inner tubular member and outer jacket can be used with delivery systems known in the art, while the preferred handle can be used with conventional inner and outer tubular member constructions. It will therefore be appreciated by those skilled in the art that yet other modifications could be made without deviating from the scope of the claims which follow.

## Claims

1. A stent delivery system, comprising:
an inner tubular member (12) having a proximal end and a distal end;
a stent (28) loaded on said distal end of said inner tubular member;
an outer jacket (14) longitudinally slidable over said inner tubular member (12) and having a proximal end and distal end; and
a handle (16) including
a stationary member (60) which defines two sides a movable member (66), and
a mechanism for moving the movable member relative to the stationary member; the mechanism comprising two knobs (68, 70) that are rotatably coupled to said stationary member, one on each of said two sides, and are mechanically coupled to said movable member such that rotation of at least one said knob causes movement of said movable member (66) relative to said stationary member (60);
wherein said proximal end of said inner tubular member (12) is fixedly coupled to said stationary member (60) and said proximal end of said outer jacket (14) is fixedly coupled to said movable member (66).

2. A stent delivery system according to claim 1, wherein:
said movable member (66) is longitudinally movable relative to said stationary member.

3. A stent delivery system according to claim 1, wherein:
said stationary member (60) defines a longitudinal axis, and said two knobs (68, 70) define an axis of rotation transverse to and vertically offset relative to said longitudinal axis.

4. A stent delivery system according to claim 1, wherein:
said two knobs (68, 70) include a periphery provided with friction-enhancing structure (106).

5. A stent delivery system according to claim 1, wherein:
said two knobs include a periphery provided with friction-enhancing material.

6. A stent delivery system according to claim 1, wherein:
said two knobs include a raised grip (108).

7. A stent delivery system according to claim 1, wherein:
said two knobs include a periphery, and an entirety of said periphery is located outside said stationary member and exposed.

8. A stent delivery system according to claim 1, further comprising:
means (14) for preventing movement of said outer jacket distally relative to said inner tubular member while permitting movement of said outer jacket proximally relative to said inner tubular member.

9. A stent delivery system according to claim 1, further comprising:
locking means for preventing movement of said movable member relative to said stationary member.

10. A stent delivery system according to claim 1, further comprising:
a cradle (200) adapted to support said stationary member.

11. A stent delivery system, according to claim 1, wherein said mechanism includes
first and second sprockets (94, 98), a toothed belt (100) extending about said first and second sprockets (94, 98), said belt being fixedly coupled to said movable member (66), and wherein the
two knobs extend at least partially outside said stationary member; said two knobs being mechanically coupled to said first sprocket (94) such that rotation of at least one said knob causes movement of said belt about said sprockets (94, 98) and longitudinal movement of said outer jacket (14) relative to said inner tubular member (12).

12. A stent delivery system according to claim 11 wherein:
said mechanism further comprises a gear (96) having first gear teeth, and
said two knobs include a periphery provided with radially-inwardly-directed second gear teeth (104) which mesh with said first gear teeth, and said gear is mechanically coupled to said first sprocket (94).

13. A stent delivery system according to claim 12, further comprising:
a stiffly resilient element (114),
wherein said mechanism includes rotatable elements with gear teeth (94, 96) and said resilient element contacts one of said rotatable elements (94) such that when said one of said rotatable elements is rotated said resilient element is caused to produce and audible clicking sound.

14. A stent delivery system, according to claim 1, further comprising:
a mechanism adapted to produce an audible sound as said outer jacket (14) is incrementally moved relative to said inner tubular member (12).

15. A method of preparing a stent delivery system for use with a stent comprising:
selecting a stent (28) of a particular length from stents having a plurality of lengths;
providing a stent delivery system in accordance with claim 1, adapting the mechanism such that regardless of said particular length of said stent, said knobs require a common movement relative to said stationary member (60) to effect retraction of said outer jacket (14) relative to said inner tubular member (12) to deploy said stent; and
loading said stent (28) on said distal end of said inner tubular member (12).

16. A method of preparing a stent delivery system for use with a stent, comprising:
selecting a stent of a particular length from stents having a plurality of lengths;
providing a stent delivery system having in accordance with claim 1, further providing a sleeve coupled to said stationary member and extending over said outer jacket, said sleeve having a distal end; wherein said outer jacket includes one of visible and tactile indicia in a length corresponding to said length of said stent of said particular length, with a proximal end of said indicia being coincident with said distal end of said sleeve; and
loading said stent on said distal end of said inner tubular member.

## Patentansprüche

1. Stentzuführsystem mit:
einem inneren rohrförmigen Element (12), das ein proximales Ende und ein distales Ende aufweist;
einem Stent (28), der an dem distalen Ende des inneren rohrförmigen Elements geladen ist;
einer äußeren Hülle (14), die in Längsrichtung über dem inneren rohrförmigen Element (12) verschiebbar ist und ein proximales Ende und ein distales Ende aufweist; und
einem Griff (16) einschließlich
eines stationären Elements (60), das zwei Seiten definiert,
eines bewegbaren Elements (66), und
eines Mechanismus zum Bewegen des bewegbaren Elements relativ zu dem stationären Element, wobei der Mechanismus zwei Knöpfe (68, 70) aufweist, die drehbar mit dem stationären Element gekoppelt sind, jeweils einer auf den zwei Seiten, und so mechanisch mit dem bewegbaren Element gekoppelt sind, dass eine Drehung des mindestens einen Knopfes eine Bewegung des bewegbaren Elements (66) relativ zu dem stationären Element (60) verursacht;
wobei das proximale Ende des inneren rohrförmigen Elements (12) fest mit dem stationären Element (60) gekoppelt ist und das proximale Ende der äußeren Hülle (14) fest mit dem bewegbaren Element (66) gekoppelt ist.

2. Stentzuführsystem nach Anspruch 1, bei dem:
das bewegbare Element (66) relativ zu dem stationären Element in Längsrichtung bewegbar ist.

3. Stentzuführsystem nach Anspruch 1, bei dem:
das stationäre Element (60) eine Längsachse definiert und die zwei Knöpfe (68, 70) eine Drehachse quer zu der Längsachse und vertikal relativ zu dieser versetzt definiert.

4. Stentzuführsystem nach Anspruch 1, bei dem:
die zwei Knöpfe (68, 70) einen Rand aufweisen, der mit einer reibungssteigernden Struktur (106) bereitgestellt ist.

5. Stentzuführsystem nach Anspruch 1, bei dem:
die zwei Knöpfe einen Rand aufweisen, der mit reibungssteigerndem Material bereitgestellt ist.

6. Stentzuführsystem nach Anspruch 1, bei dem:
die zwei Knöpfe einen erhöhten Griff (108) aufweisen.

7. Stentzuführsystem nach Anspruch 1, bei dem:
die zwei Knöpfe einen Rand aufweisen und eine Gesamtheit des Rands außerhalb des stationären Elements angeordnet und exponiert ist.

8. Stentzuführsystem nach Anspruch 1, ferner mit:
einem Mittel (14) zum Vorbeugen gegen eine Bewegung der äußeren Hülle in distaler Richtung relativ zu dem inneren rohrförmigen Element, während es eine Bewegung der äußeren Hülle in proximaler Richtung relativ zu dem inneren rohrförmigen Element zulässt.

9. Stentzuführsystem nach Anspruch 1, ferner mit:
einem Verriegelungsmittel zum Vorbeugen gegen eine Bewegung des bewegbaren Elements relativ zu dem stationären Element.

10. Stentzuführsystem nach Anspruch 1, ferner mit:
einem Gestell (200), das angepasst ist, das stationäre Element zu unterstützen.

11. Stentzuführsystem nach Anspruch 1, bei dem der Mechanismus aufweist
ein erstes und zweites Ritzel (94, 98),
einen Zahnriemen (100), der sich um das erste und zweite Ritzel (94, 98) erstreckt, wobei der Riemen fest mit dem bewegbaren Element (66) gekoppelt ist und sich die
zwei Knöpfe zumindest teilweise von dem stationären Element nach außen erstrecken, wobei die zwei Knöpfe mechanisch mit dem ersten Ritzel (94) gekoppelt sind, sodass eine Drehung des mindestens einen Knopfes eine Bewegung des Riemens um die Ritzel (94, 98) und eine Längsbewegung der äußeren Hülle (14) relativ zu dem inneren rohrförmigen Element (12) verursacht.

12. Stentzuführsystem nach Anspruch 11, bei dem:
der Mechanismus ferner ein Zahnrad (96) mit ersten Zahnradzähnen aufweist, und
die zwei Knöpfe einen Rand aufweisen, der mit radial nach innen gerichteten zweiten Zahnradzähnen (104) bereitgestellt sind, die mit den ersten Zahnradzähnen kämmen, und das Zahnrad mechanisch mit dem ersten Ritzel (94) gekoppelt ist.

13. Stentzuführsystem nach Anspruch 12, ferner mit:
einem steifen Federelement (114),
wobei der Mechanismus drehbare Elemente mit Zahnradzähnen (94, 96) aufweist und das federnde Element mit einem der drehbaren Elemente (94) in Kontakt ist, sodass, wenn das eine der drehbaren Elemente gedreht wird, verursacht wird, dass das Federelement ein hörbares Klickgeräusch erzeugt.

14. Stentzuführsystem nach Anspruch 1, ferner mit:
einem Mechanismus, der angepasst ist, ein hörbares Geräusch zu erzeugen, während die äußere Hülle (14) schrittweise relativ zu dem inneren rohrförmigen Element (12) bewegt wird.

15. Verfahren zum Vorbereiten eines Stentzuführsystems für eine Verwendung mit einem Stent, das umfasst:
Auswählen eines Stents (28) bestimmter Länge aus Stents mit einer Vielzahl von Längen;
Bereitstellen eines Stentzuführsystems in Übereinstimmung mit Anspruch 1,
Anpassen des Mechanismus, sodass unabhängig von der bestimmten Länge des Stents die Knöpfe eine gemeinsame Bewegung relativ zu dem stationären Element (60) benötigen, um zum Einsetzen des Stents ein Zurückziehen der äußeren Hülle (14) relativ zu dem inneren rohrförmigen Element (12) zu bewirken; und
Laden des Stents (28) an das distale Ende des inneren rohrförmigen Elements (12).

16. Verfahren zum Vorbereiten eines Stentzuführsystems für eine Verwendung mit einem Stent, das umfasst:
Auswählen eines Stents einer bestimmten Länge aus Stents mit einer Vielzahl von Längen;
Bereitstellen eines Stentzuführsystems in Übereinstimmung mit Anspruch 1,
ferner Bereitstellen einer Hülse, die mit dem stationären Element gekoppelt ist und sich über die äußere Hülle erstreckt, wobei die Hülse ein distales Ende aufweist, die äußere Hülle eine sichtbare oder hörbare Anzeige in einer Länge aufweist, die mit der Länge des Stents bestimmter Länge korrespondiert, mit einem proximalen Ende der Anzeige mit dem distalen Ende der Hülse zusammen fallend; und
Laden des Stents an das distale Ende des inneren rohrförmigen Elements.

## Revendications

1. Système de pose d'endoprothèse vasculaire, comprenant :
un élément tubulaire interne (12) ayant une extrémité proximale et une extrémité distale ;
une endoprothèse vasculaire (28) chargée sur ladite extrémité distale dudit élément tubulaire interne ;
une gaine externe (14) pouvant coulisser longitudinalement sur ledit élément tubulaire interne (12) et ayant une extrémité proximale et une extrémité distale ; et
une poignée (16) comportant
un élément fixe (60) qui définit deux côtés
un élément mobile (66), et
un mécanisme pour déplacer l'élément mobile par rapport à l'élément fixe ; le mécanisme comprenant deux boutons (68, 70) qui sont couplés en rotation audit élément fixe, l'un sur chacun desdits deux côtés, et sont mécaniquement couplés audit élément mobile de sorte que la rotation d'au moins ledit bouton entraîne le mouvement dudit élément mobile (66) par rapport audit élément fixe (60) ;
dans lequel ladite extrémité proximale dudit élément tubulaire interne (12) est couplée de manière fixe audit élément fixe (60) et ladite extrémité proximale de ladite gaine externe (14) est couplée de manière fixe audit élément mobile (66).

2. Système de pose d'endoprothèse vasculaire selon la revendication 1, dans lequel :
ledit élément mobile (66) est longitudinalement mobile par rapport audit élément fixe.

3. Système de pose d'endoprothèse vasculaire selon la revendication 1, dans lequel :
ledit élément fixe (60) définit un axe longitudinal,
et lesdits deux boutons (68, 70) définissent un axe de rotation transversal audit axe longitudinal et décalé verticalement par rapport à celui-ci.

4. Système de pose d'endoprothèse vasculaire selon la revendication 1, dans lequel :
lesdits deux boutons (68, 70) comportent une périphérie pourvue d'une structure d'augmentation de frottement (106).

5. Système de pose d'endoprothèse vasculaire selon la revendication 1, dans lequel :
lesdits deux boutons comportent une périphérie pourvue d'un matériau d'augmentation de frottement.

6. Système de pose d'endoprothèse vasculaire selon la revendication 1, dans lequel :
lesdits deux boutons comportent un élément de préhension surélevé (108).

7. Système de pose d'endoprothèse vasculaire selon la revendication 1, dans lequel :
lesdits deux boutons comportent une périphérie, et la totalité de ladite périphérie est située à l'extérieur dudit élément fixe et exposée.

8. Système de pose d'endoprothèse vasculaire selon la revendication 1, comprenant en outre :
un moyen (14) pour empêcher un mouvement de ladite gaine externe de manière distale par rapport audit élément tubulaire interne tout en permettant un mouvement de ladite gaine externe de manière proximale par rapport audit élément tubulaire interne.

9. Système de pose d'endoprothèse vasculaire selon la revendication 1, comprenant en outre :
un moyen de verrouillage pour empêcher un mouvement dudit élément mobile par rapport audit élément fixe.

10. Système de pose d'endoprothèse vasculaire selon la revendication 1, comprenant en outre :
un berceau (200) adapté pour supporter ledit élément fixe.

11. Système de pose d'endoprothèse vasculaire selon la revendication 1, dans lequel ledit mécanisme comporte
des premier et deuxième pignons (94, 98),
une courroie dentée (100) s'étendant autour desdits premier et deuxième pignons (94, 98), ladite courroie étant couplée de manière fixe audit élément mobile (66), et dans lequel les deux boutons s'étendent au moins partiellement à l'extérieur dudit élément fixe ; lesdits deux boutons étant mécaniquement couplés audit premier pignon (94) de sorte que la rotation d'au moins ledit bouton entraîne un mouvement de ladite courroie autour desdits pignons (94, 98) et un mouvement longitudinal de ladite gaine externe (14) par rapport audit élément tubulaire interne (12).

12. Système de pose d'endoprothèse vasculaire selon la revendication 11, dans lequel :
ledit mécanisme comprend en outre un engrenage (96) ayant une première denture, et
lesdits deux boutons comportent une périphérie pourvue d'une deuxième denture dirigée radialement vers l'intérieur (104) qui s'engrène avec ladite première denture, et ledit engrenage est mécaniquement couplé audit premier pignon (94).

13. Système de pose d'endoprothèse vasculaire selon la revendication 12, comprenant en outre :
un élément rigidement élastique (114),
dans lequel ledit mécanisme comporte des éléments rotatifs avec dentures (94, 96) et ledit élément élastique entre en contact avec l'un desdits éléments rotatifs (94) de sorte que, lorsque ledit élément parmi lesdits éléments rotatifs est mis en rotation, ledit élément élastique soit amené à produire un clic audible.

14. Système de pose d'endoprothèse vasculaire selon la revendication 1, comprenant en outre :
un mécanisme adapté pour produire un son audible à mesure que ladite gaine externe (14) se déplace progressivement par rapport audit élément tubulaire interne (12).

15. Procédé de préparation d'un système de pose d'endoprothèse vasculaire pour une utilisation avec une endoprothèse vasculaire comprenant le fait :
de sélectionner une endoprothèse vasculaire (28) d'une longueur particulière à partir d'endoprothèses vasculaires ayant une pluralité de longueurs ;
de fournir un système de pose d'endoprothèse vasculaire selon la revendication 1, d'adapter le mécanisme de sorte qu'indépendamment de ladite longueur particulière de ladite endoprothèse vasculaire, lesdits boutons nécessitent un mouvement commun par rapport audit élément fixe (60) pour effectuer une rétraction de ladite gaine externe (14) par rapport audit élément tubulaire interne (12) pour déployer ladite endoprothèse vasculaire ; et
de charger ladite endoprothèse vasculaire (28) sur ladite extrémité distale dudit élément tubulaire interne (12).

16. Procédé de préparation d'un système de pose d'endoprothèse vasculaire pour une utilisation avec une endoprothèse vasculaire, comprenant le fait :
de sélectionner une endoprothèse vasculaire d'une longueur particulière à partir d'endoprothèses vasculaires ayant une pluralité de longueurs ;
de fournir un système de pose d'endoprothèse vasculaire selon la revendication 1, de fournir en outre un manchon couplé audit élément fixe et s'étendant sur ladite gaine externe, ledit manchon ayant une extrémité distale ; où ladite gaine externe comporte l'un des indices visibles et tactiles dans une longueur correspondant à ladite longueur de ladite endoprothèse vasculaire de ladite longueur particulière, avec une extrémité proximale desdits indices étant en coïncidence avec ladite extrémité distale dudit manchon ; et
de charger ladite endoprothèse vasculaire sur ladite extrémité distale dudit élément tubulaire interne.
